# EUROPEAN PATENT SPECIFICATION

(11) **EP 3 188 786 B1**
(45) Date of publication and mention of the grant of the patent: **22.04.2020**
(21) Application number: 15756647.2
(22) Date of filing: 28.08.2015
(51) Int. Cl.: A61M 25/02, A61B 17/068, A61M 25/00, A61M 25/01, A61M 27/00

(54) **TUBE FIXATION DEVICE**
ROHRFIXIERUNGSVORRICHTUNG
DISPOSITIF DE FIXATION DE TUBE

(30) Priority: 02.09.2014 SE 1451019; 28.04.2015 SE 1550511; 29.04.2015 SE 1550518
(43) Date of publication of application: 12.07.2017
(73) Proprietor: Helse Stavanger HF, 4068 Stavanger (NO)
(72) Inventor: OVELAND, Nils Petter, N-4027 Stavanger (NO)
(74) Representative: Brann AB
(86) International application number: PCT/EP2015/069743
(87) International publication number: WO 2016/034508

(56) References cited:
- US-A- 4 857 058
- US-A1- 2004 204 685
- US-A1- 2007 198 026
- US-A1- 2007 265 571

## Description

### TECHNICAL FIELD

The present invention relates to the field of medical devices for use in surgical procedures, in particular medical devices for aiding in procedures involving draining of gases and fluids from body cavities in a trauma setting and critical care settings.

### BACKGROUND OF THE INVENTION

Trauma is one of the leading causes of death worldwide (5.1 million deaths in 2010) and can be characterized as a global epidemic because it accounts for one in every 10 deaths. Patients with multiple blunt injuries are far more common in civilian practice, but both penetrating and blunt trauma present significant challenges to national health care systems and necessitate a policy action to prevent them. Chest injury is the direct cause of death in 25% of blunt trauma victims and a contributing factor in up to another 50% of trauma deaths, which can be explained by the large number of motor vehicle crashes and falls. Serious consequences of chest trauma are pneumothorax and/or haemothorax, potential life-threatening conditions that sometimes requires immediate treatment with chest drainage (i.e. thoracostomy) to prevent patient death. Between 18% and 40% of patients sustaining thoracic trauma can be treated with chest tubes alone. A thoracotomy (i.e. a larger incision commonly used to gain access to organs within the chest) will only be required for between 3% and 9% of patients. Even among those with penetrating trauma, only 14% of stab wounds and between 15% and 20% of gunshot to the chest require thoracotomy. In conclusion, most chest trauma patients can be managed with chest tubes.

Chest tube thoracostomy is a common and very useful therapeutic procedure. It is indicated in pneumothorax with or without tension, traumatic haemothorax, haemopneumothorax, etc. However, it is not without risk, especially when aggressively used in trauma patients. After placement, the chest tube position may be altered if the drain is not secured tightly to the chest. Different techniques have been described for anchoring chest tubes, but have various disadvantages.

Previous art has shown that a surgical tape can be used to cover the incision and anchor the tube close to the chest wall and an omental tag of tape can hold the tube close to the chest wall, allowing some motion of the tube without kinking. Additionally, the accessory tubing can be pinned to the hospital bed for more security. However, the problem with using surgical tape in trauma patients is that blood and sweat on the skin often prevent a decent grip. A more common technique is to anchor the drain to the chest with sutures. There are numerous ways of doing this. Still, the technique requires a suture-kit, clinical experience in stitching and is time-consuming.

In general chest tube complications are categorized as insertional, positional or infective. More specifically, pain, vascular injury, improper positioning of the tube, inadvertent tube removal, postremoval complications, longer hospital stays, empyema and pneumonia have been reported in up to 30% of cases.

Today there are several solutions on the market. One "homemade" solution is to use a defibrillation pad and tape it around the chest tube. Commercial solutions of surgical tape are similar and use some kind of adhesive dressing that surrounds the chest drain. In interventional radiology smaller chest catheters are often inserted into the pleural cavity and fixated with some kind of locking mechanism. Still, the most common fixation method is to suture the drain to the chest. Examples of tube fixation devices are also described in US 2014/0031753, WO 9325264, CN 202892621, US 2006/02572, and GB 2160776. A further example of a tube fixation element according to the preamble of claim 1 is disclosed in document US 2007/198026.

If the patient is stable and the chest drain is inserted at the hospital under optimal conditions different surgical tapes will be sufficient to secure the drain. Still, if the patients moves and/or pulls the drain it may very well be dislodged. In trauma patients the surgical tape will often not fasten due to blood and sweat on the skin. This allows for inadvertent tube removal and considerable leakage of air around the chest tube. An addition problem is kinking of the drain underneath tape/dressings. This may remain unrecognized by health care providers until the patients get unstable. Suturing techniques requires experience, a suture-kit and is above all time-consuming. The latter is a problem in unstable trauma patients in rapid need of transportation to hospital. Therefore, tube thoracostomy is not performed or delayed in some critical situations.

In conclusion, sutures are an adequate method to secure chest tubes in injured patients, but it is time-consuming, requires a suture-kit and clinical experience/skills.

Hence, there is still a need in the art to provide improved medical devices for providing an easier and more straightforward manner to perform drainage of fluids and gases from mammalian bodies, especially in time-critical situations and in non-hospital settings.

### SUMMARY OF THE INVENTION

A tube fixation device is presented herein which solves or at least mitigates the above cited problems by providing a tube fixation device for maintaining a tube inserted into a mammalian body in a releasably fixated position, wherein said tube fixation device comprises a tube receiving element adapted to receive said tube; a fastening element arranged in connection to said tube receiving element and wherein said fastening element is adapted to releasably fixate said tube when said tube is arranged in said tube receiving element; and a supportive element attached to said tube receiving element and adapted to be positioned on a surface area of said mammalian body. The supportive element comprises a layered structure, wherein the layered structure comprises at least a ductile layer comprising a ductile material. The ductile layer is configured to be shapeable to conform to a three-dimensional shape of the surface area on the mammalian body. In some embodiments, the ductile layer is configured to essentially maintain the obtained three-dimensional shape.

In addition, the present disclosure relates to a kit comprising a tube fixation device, and further comprising at least a device for applying surgical staples or stitches, a tube and instructions for use.

The present disclosure also concerns a method for maintaining a tube inserted into a mammalian body in a releasably fixated position comprising arranging said tube into a device and thereafter fastening said tube through said fastening element.

Notably, the device as presented herein is particularly suitable for fixation of a chest tube which has been inserted into a mammalian body.

### BRIEF DESCRIPTION OF THE DRAWINGS

Figures 1a and 1b show a schematic perspective of a side view of a tube fixation device.
Figures 2a and 2b show a schematic perspective of a top view of a tube fixation device.
Figure 3 shows a bottom view of a supportive element (adhesive layer) of a tube fixation device.
Figure 4 shows a side view of a tube fixation device.
Figure 5 shows a cross-section of a tube receiving element, a fastening element and a supportive element of a tube fixation device.
Figures 6a-b show perspective views of from a top side and bottom side, respectively, of a tube fixation device.
Figures 7a-b show a schematic side view and a top view, respectively, of a tube fixation device.
Figures 8a-d shows different views of another embodiment of the tube fixation device.
Figure 9 shows an illustration of insertion of a tube for subsequent fixation with a tube fixation device.
Figures 10a and 10b show two different examples of a kit comprising a tube fixation device.
Figure 11 shows a tube fixation device when attached in position.
Fig 12 shows another tube fixation device.
Figure 13a and 13b show a ductile layer of a supportive element in a tube fixation device.
Figure 14 shows another tube fixation device.

### DETAILED DISCLOSURE OF THE INVENTION

The present disclosure concerns a tube fixation device (1) for maintaining a tube (2) inserted into a mammalian body during a drainage procedure. The tube fixation device comprises a tube receiving element adapted to receive said tube; a fastening element arranged in connection to said tube receiving element and wherein said fastening element is adapted to releasably fixate said tube when said tube is arranged in said tube receiving element; and a supportive element attached to said tube receiving element and adapted to be positioned on a specified surface area of said mammalian body. The supportive element comprises a layered structure, wherein the layered structure comprises at least a ductile layer comprising a ductile material. The supportive element can comprise one or several further layers, such as an absorbent layer, a flexible layer, and/or an adhesive layer. In some embodiments the supportive element can also comprise further layers, such as a sealing layer and/or an anti-infective layer. These will be described in detail below.

The device allows anchoring of tubes, such as chest tubes to the patient's chest without the need of time-consuming and technical challenging suture techniques. Its ease to use will save time in critical situations and the device is designed to be applied in a quick and easy manner. The fixation of the tube avoids kinking thereof, and use of staples or stitches and/or an adhesive further provides a more time efficient manner of attaching the device.

Blood and sweat from the skin will be removed by components of the dual core system of a preferred embodiment of the device (1). Herein, a dual core system refers to a layered structure including an absorbent layer and ductile layer forming part of the supportive element (5) for the tube fixation device (1). The dual core is capable of absorbing blood, sweat and other bodily fluids.

The ductile layer may comprise a plate, such as a metal or plastic plate, for better fitting of the device to a part of the body, such as the chest, of a subject. Herein, the word ductile refers to any material which contributes the characteristics of the ductile layer, and thereby the supportive element, being able to conform to a three-dimensional shape under certain pressure. In some embodiments, a ductile layer further essentially maintains the resulting shape after pressure is removed. Or, phrased differently, the ductile layer is adapted to be shapeable under certain applied pressure to conform to, i.e. follow the contours of, the three-dimensional shape of the surface area on the mammalian body. Further, the ductile layer will in some embodiments also essentially maintain the obtained three-dimensional shape after the pressure is removed. In other words, a ductile layer in a tube fixation device will allow a user to apply the tube fixation device by pressing down, e.g. by pressing with his/her hands, on the supportive element, such that the ductile layer, and thus the supportive element, will adapt to the three-dimensional shape of the underlying body part, e.g. a chest area. When the applied pressure is removed, the ductile layer, and thus the supportive element, will essentially maintain the three-dimensional shape of the underlying body part. Both these features contribute separately to providing pressure on the underlying mammalian body.

Further, if needed, by applying pressure to the supportive element again, the ductile layer can be reshaped to a new three-dimensional shape.

Thus, the ductile layer ensures a tight interaction between the supportive element and the surface application area on the mammalian body, both during application of the device, and throughout the time period during which the chest tube fixation device is maintained on the mammalian body. The ductile layer also provides for a durable supportive element, which is an advantage under harsh conditions, such as applying a chest tube fixation device at an accident site, under emergency transport or in a military field setting.

The ductile layer may be a layer with a surface area corresponding to essentially a majority of the surface area of the supportive element, such that the major part of the supportive element can be shaped to follow the curvature of the underlying body.

As an alternative, the ductile layer may be layer with a surface area corresponding to a smaller surface area than the supportive element, i.e. a part of the surface of the supportive element, wherein the surface area of the ductile layer is sufficient enough to contribute the characteristics of the ductile layer as described above, i.e. allowing the supportive element to at least conform to a three-dimensional shape under certain pressure.

In one embodiment, a tube fixation device is also provided with an absorbent layer as one of the layers in the supportive element. The absorbent layer may be adapted to be arranged below or under the ductile layer in relation to said mammalian body, i.e. closer to the skin of the patient than the ductile layer. The absorbent layer may also be arranged above or over the ductile layer. As a further alternative, one or more absorbent layers may be arranged both above and below the ductile layer in a supportive element. The absorbent layer (24) provides for the absorption of any body fluids excreted from or present around the entry area of the tube drainage. In addition, a supportive element comprising both a ductile layer and an absorbent layer provides for effectively lessening or even preventing bleeding from the skin incision, based on the absorbing layer absorbing fluids and the compressive force due to the ductile layer. In such an embodiment, the ductile layer and the absorbent layer preferably form layers of essentially the same surface area within the supportive element, and preferably the size and shape of the respective surface areas correspond to each other.

The ductile layer may be essentially or completely impermeable to any fluids and other contaminants. This arrangement will prevent any fluids from soaking through all the layers of the supportive element. Thus, any body fluids stemming from the wound site will be contained under the ductile layer. Likewise, any fluid or other contaminants, such as rain, dirt, bacteria etc., will be kept away from the wound site. Hence, an impermeable ductile layer has the advantage of being an important hygienic precaution to protect the users of the device to get in contact with foreign body fluids, and to preserve sterility of the wound site.

In some embodiments, a flexible layer is arranged in the supportive element. This flexible layer is then arranged above or over the other layers, such that the flexible layer provides both overall flexibility of the supportive device, and also protects the other layers and the wound site. The flexible layer may be made of a waterproof and/or wear-proof material, such that the wound site and the underlying ductile and absorbent layers are sealed off and protected from rain, wind, dirt, bacteria etc.; hence contamination of the wound site is reduced or avoided, similarly to the ductile layer being impermeable, as described above. The flexible layer may cover at least the same surface area as the ductile layer and, if present, also at least the same surface area as the absorbent layer. A flexible layer may also be larger in surface area than a ductile layer, especially if the ductile layer is smaller in surface area than the other layers.

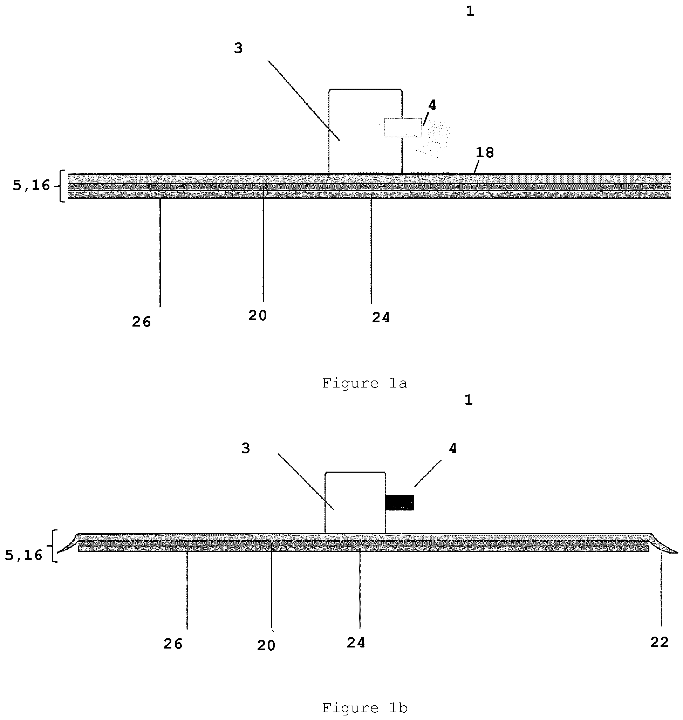

The flexible layer can further be provided with a rim extending beyond the other layers, which thus allows for fastening of the device to the body by stitches or staples through the rim (22) of the flexible layer (see Figure 1b and below). This provides for quick and secure attachment of the device to the patient. In such an embodiment, the flexible layer thus covers a surface area extending beyond the surface area of any underlying layers.

The tube receiving element (3) and the supportive element (5) may be tightly attached to each other, which provides for sealing of any leakage of air or fluid outside the tube receiving element (3). In one embodiment, the tube receiving element is continuously connected with the ductile layer, either by being molded as one piece, or welded or glued or clipped or screwed together such that they form a continuous part.

This is further described below. Further, sealing within the tube receiving element (3), i.e. around the tube (2) inside the tube receiving element (3), will be further addressed below.

Furthermore, usage of staples to secure the tube fixation device (1) to the skin of the patient may further prevent the tube (2) from dislodging when the patient moves or is being transported. This is especially true when also using an adhesive layer, as described below. Therefore, the tube fixation device (1) is particularly useful to use in emergency clinical or pre-hospital situations as it is faster than conventional procedures. The time required is considerable less than the techniques available today. Preliminary animal trials indicate that the time required in one third of the normal stitching technique (Oveland et al, SEARCH 2013).

In the below the device will be further described with reference to the figures. Throughout the figures the same or similar functions/items have been given the same reference signs.

Figure 1a illustrates a side view of a tube fixation device (1) for maintaining a tube (2) inserted into a mammalian body in a releasably fixated position, said tube fixation device (1) comprising a tube receiving element (3) adapted to receive said tube (2) (not shown), a fastening element (4) arranged in connection to said tube receiving element (3) and wherein said fastening element (4) is adapted to releasably fixate said tube (2) when said tube (2) is arranged in said tube receiving element (3) and a supportive element (5) attached to said tube receiving element and adapted to be positioned on said mammalian body. In the present context, the tube is fixated when it is secured in position in the tube receiving element (3), however it may be slightly movable when in position as long as it fulfils its purpose as presented herein, i.e. does not move in a longitudinal direction. Fig 1b illustrates the embodiment where a rim (22) of the top flexible layer (18) extends beyond the edges of the below layers (20, 24, 26), as indicated above.

In this embodiment, the fastening element (4) is configured to apply a radially directed fastening pressure in relation to said tube (2). Such a force may be applied e.g. by carefully pushing or squeezing the tube receiving element (3) against the tube (2) thereby securing the tube (2) against the walls of the tube receiving element (3). The tube receiving element (3) may have any suitable structure still allowing the tube (2) to be securely releasably fixated against the tube receiving element (3), such as a cylindrical (pipe-shaped) or rectangular shape, preferably an elongated shape. The tube receiving element (3) may be made from any material suitable for the purpose such as plastic, metal material or made of a fabric. Examples of suitable materials for the respective parts of the device are further exemplified herein. As an example, if made of fabric, the tube receiving element (3) may contain threads or strips allowing for tightening of the tube receiving element (3) around the tube (2) upon receiving the tube, i.e. the fastening pressure is a squeezing or gripping pressure equally spread around the circumference of the tube receiving element. In another embodiment, the fastening element may be a clamp that applies a similar circumferential squeezing or gripping pressure. In another embodiment the fastening element may be locked by rotating clockwise to apply the same circumferential squeezing or gripping pressure, and anticlockwise to unlock, i.e. to reduce the squeezing or gripping pressure.

In a further embodiment, the tube receiving element (3) may be transparent for easy visibility of the tube when present in the tube receiving element (3). This is further described below.

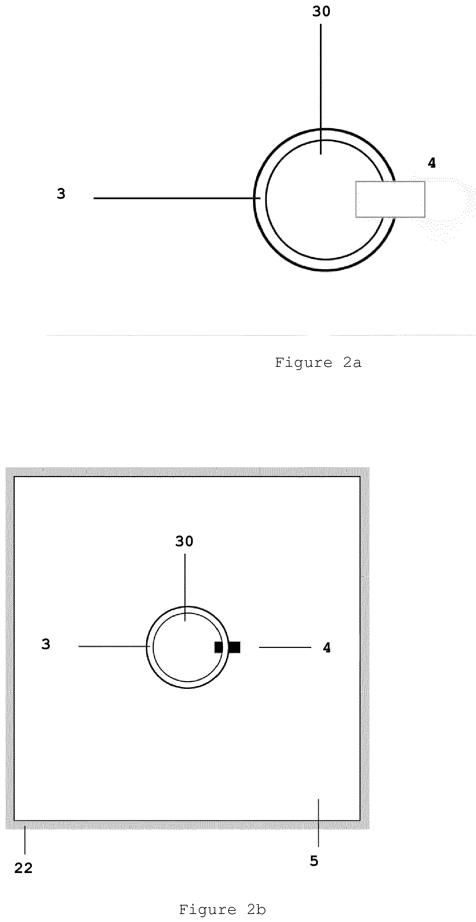

Figure 2a illustrates a cross-section of a device (schematic) wherein the tube receiving element (3) is illustrated as having a cylindrical hollow (30) shape, even if it is not limited to having such a shape, to therein receive the tube (2). The tube receiving element (3) may also have an opening for a fastening element (4), wherein the fastening element may be adapted to securely releasably fixate the tube by protruding through the opening into the tube receiving element (3) and thereby compressing the tube against a wall of the tube receiving element (3). The tube is secured with a suitable pressure but without damaging the tube (2). The tube (2) may then be released from the tube fixation device (1) by withdrawing the fastening element (4) through the opening in the tube receiving element (3). The fastening element may be adapted to be slidably fixated by friction in an opening of the tube receiving element, or be provided with threads, barbs or the like to attach the fastening element to the tube receiving element (3). The fastening element (4) may be a plug or a wedge. Figure 2b illustrates the device (1) including the supportive element (5).

Further advantages of the device of the present disclosure include reduction of spontaneous movement of the tube (2), e.g. the chest tube due to patient movement. This will be achieved by the drain or the tube being tightly fastened through the fastening element (4), such as a screw-handle (see figure). The technique according to the present disclosure is also a quick and a more easy technique in trauma patients in acute need of chest drainage, especially in challenging settings such as pre-hospital, battle-field, remote areas and emergency departments.

The supportive element of a tube fixation device may comprise a ductile layer (20), an absorbent layer (24) and a flexible layer (18) as described above (see e.g. Figure 1a and 1b). In addition, the supportive element (5) may further comprise an adhesive layer (26) comprising an adhesive material, such as a tape. The adhesive layer is arranged below or under the absorbent layer in relation to said mammalian body, i.e. closest to the skin of the patient. The adhesive layer provides for improved attachment of the fixation device to the mammalian body.

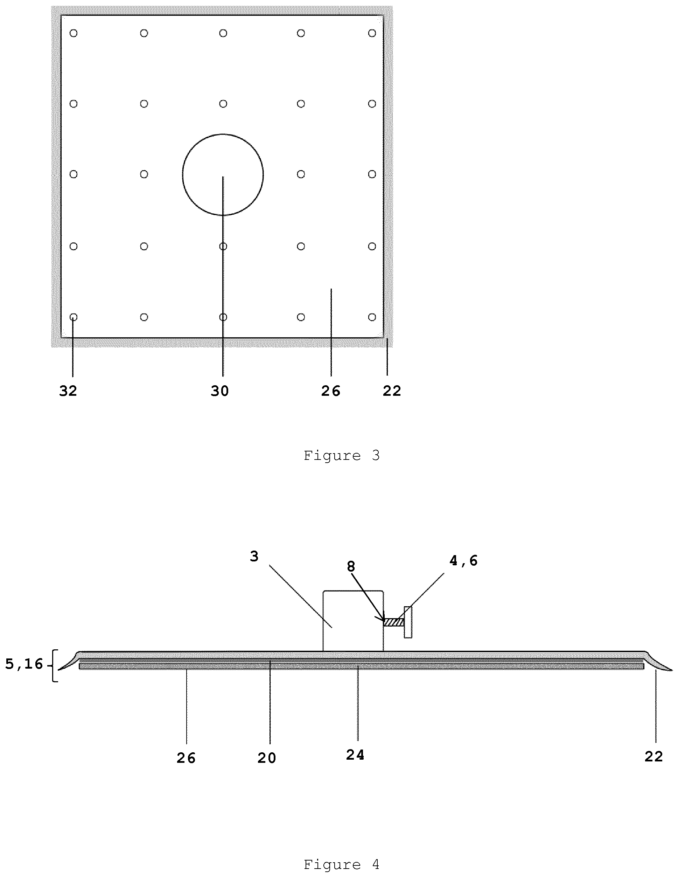

Figure 3 shows a bottom view of a supportive element (5) of a further embodiment of the tube fixation device, wherein an aperture (30) has been made through the supportive element (5) for the tube (2) to pass through, and wherein an adhesive layer (26) is shown having pores/micropores (32) allowing for bodily fluids to pass through the adhesive layer (26).

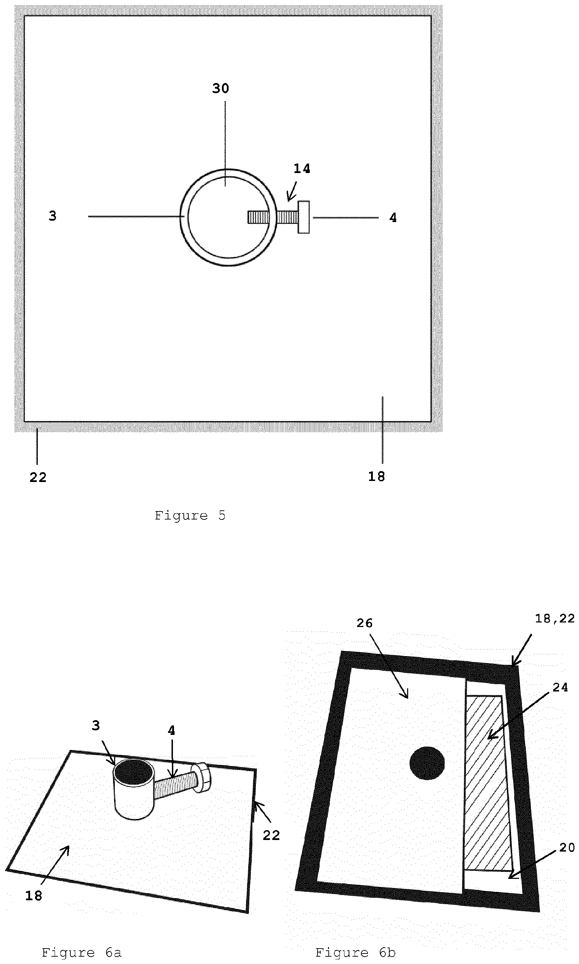

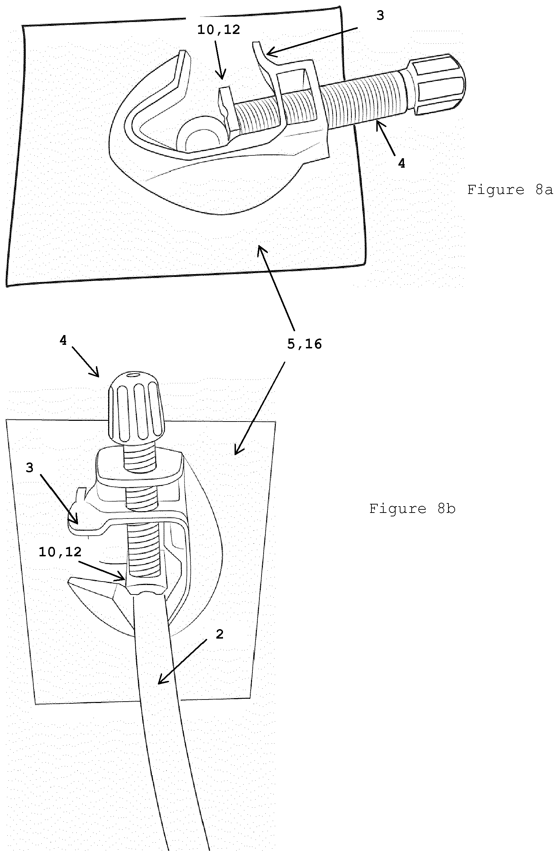

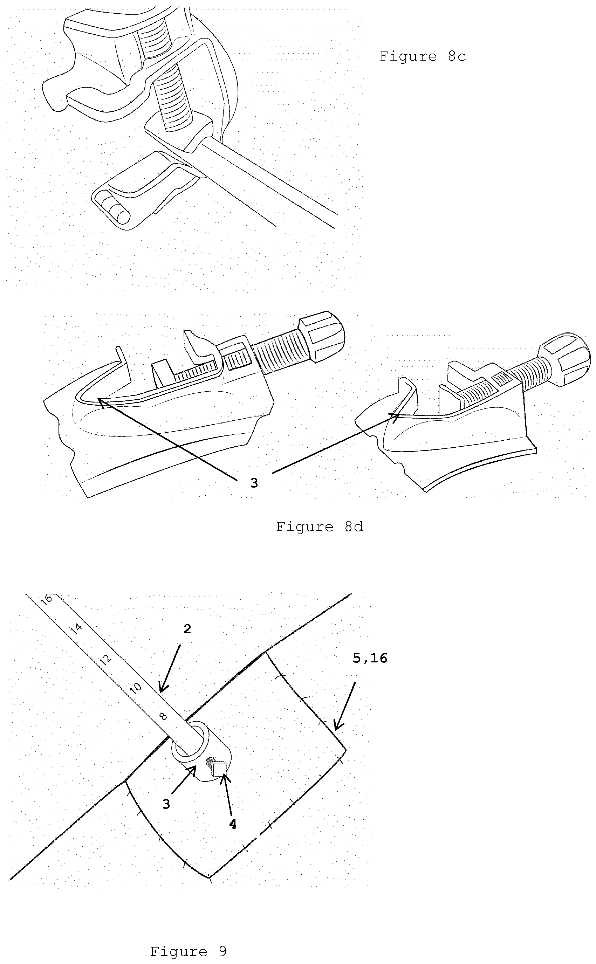

In one embodiment said fastening element (4) comprises an elongated threaded element (6) and said tube receiving element contains a threaded opening (8) adapted to receive and rotatably attach said threaded element (6) to said tube receiving element (3). Said threaded element (6) may be a screw, said screw optionally being arranged with a handle, or a similar structure, wherein the screw is adapted to fixate the tube in the tube receiving element (3) by rotating the screw via the threaded opening (8) and thereby compressing the tube against the wall of the tube receiving element (3). An end of said threaded element (6) may also comprise an engagement member (10) for engaging and fixating said tube (2). An example of such an embodiment of the tube fixation device is illustrated in figures 4 and 5. Said engagement member (10) may have a flattened surface (12) for engaging and fixating said tube (2). This is illustrated in an embodiment of Figure 8, but the feature is not limited thereto. As shown in figure 4 and 5, said tube receiving element may have the shape of an elongated hollow pipe (3). The pipe (3) may be made of any suitable material as mentioned herein. Said elongated hollow pipe (3) may be arranged with an aperture (14) for receiving said fastening element (3). In one embodiment, said tube receiving element (3) has a clamp-like structure and said threaded element is in the form of a screw with a handle.

Further, in other embodiments, the pipe (3) is a round cylinder with a side aperture where a screw with a handle is attached. By tightening the screw, the tubes that goes through the pipe and plate of the device is therewith fastened. The tip of the screw presses the chest tube against the wall inside the pipe (3).

In other embodiments, a pipe or a similar structure could also be used, but the mechanism may be that the screw is tightened to compress the chest tube against another inner wall of the tube receiving element. The tip, i.e. the engagement member, of the screw may also be in a shape that fits the round shape of the tubes. The advantages of a releasable fastening element are that it is possible to unscrew, reposition and replace the tubes if necessary.

The materials of the pipe may be metal or hard plastic, or transparent so the number readings on the tubes are visible.

As an alternative to using a fastening element with a radially directed fastening pressure, it is also conceivable that a circumferential gripping pressure be used. Such solutions, as will be described below, are understood to be able to replace or be used in combination with the radially directed fastening pressure solutions described herein. Thus, in the figures, any fastening element (4) could also be arranged around the entire circumference or parts of the circumference of the tube receiving element.

One such alternative fixation mechanism for the tube (2) is to have a tube receiving element (3), such as a pipe, without a fastening element (4) in the form of a screw on the side. This embodiment corresponds to e.g. Figure 1a wherein the fastening element (4) instead is arranged around the circumference of the pipe (3), and not just at one position as shown in the figure. Turning the tube receiving element (3) or the pipe in a clockwise direction will decrease the diameter of the aperture and finally compress the tube (chest tube) around the whole circumference, i.e. exerting a circumferential squeezing or gripping pressure. The process will be reversed when the pipe (3) is turned in the anticlockwise direction i.e. the diameter will increase and the chest tube unfixed to do any adjustments.

As an alternative to, or used in combination with a rotational lock as described above, a pull-lock can be arranged. In such an embodiment, the tube receiving element is arranged with a locking mechanism where the diameter is decreased when the tube receiving element is pulled up or away from the supportive element, such that the tube is fastened. When the tube receiving element is pushed downward, the diameter is increased, and the tube released. It is also conceivable that the mechanism is reserved, i.e. that when the tube receiving element is pushed down or towards the supportive element the diameter is decreased and the tube is fastened in the tube receiving element.

Another alternative is wherein said tube receiving element (3) is a soft pipe not made of metal or plastic, but of fabric or similar material. Incorporated in the fabric may be a thread or strips. The tube fixation device slides over the chest tube, through the central hole in the plate and the soft fabric pipe/protrusion. Then the thread or strips are tightened around the circumference of the chest tube to exert a circumferential or gripping pressure.

As mentioned above and illustrated in figures 1,4, and 7, a tube fixation device (1) comprises a supportive element (5) comprising a layered structure (16). The supportive element (5) is adapted to provide a supportive structure for the tube receiving element (3) and the fastening element (4), and in preferred embodiments, to allow for the absorbing of bodily fluids, such as blood and sweat. Further, in some embodiments, a flexible layer allows for fastening of the device to the body by stitches or staples through the rim (22) of the flexible layer. Said layered structure (16) may comprise at least two layers, such as three, four, five or more layers.

Said layered structure (16) comprises a ductile layer (20) comprising a ductile material, such as a plastic or metal material. Non-limiting examples of materials that can be used are shape-retaining plastic materials (e.g. PA2200 or a similar flexible plastic polymer) or a bendable and strong metal material such as Alumide® or an equally bendable metal alloy (reference www.shapeways.com). A preferred material is a thin sheet of a metal alloy such as an aluminum alloy, preferably with a thickness in the range of about 0,5-5 mm, more preferably in the range of about 1,0-3,0 mm. However, any choice of material is within the scope of the present disclosure, as long as the ductile layer (20) provides a bendable layer which can be shaped to the curvatures of the body, such as the chest wall, on application of a pressure as described above. Further, any material providing the same function can be used. The ductile material has the characteristics in that it is bendable in all directions so that it can be fitted to the shape of a body. In addition, the ductile material is preferably a light-weight, strong material and capable of maintaining its own shape after being shaped by hand by applying pressure.

In addition, the material in the ductile layer should preferably essentially not be affected by extreme temperatures, especially relating to the bendability and shapeability characteristics, as well as being capable of maintaining its shape when in position.

The ductile layer may be essentially radiolucent, i.e. does not cause any major disturbances or affect the picture quality to any discernable degree when imaging techniques are used, e.g. X-ray or CT (Computed Tomography). Thus, an advantage of the ductile layer being radiolucent is that in situations when an imaging technique, such as CT or X-ray imaging, needs to be used on the subject having the fixation device attached thereto, picture quality is essentially unaffected. In certain imaging procedures, metal materials will disrupt or distort the image and/or cause other problems, however the inventor has shown that by using the preferred material described above, e.g. a thin sheet of aluminum alloy or the like, the ductile layer will not affect either an X-ray image or a CT image to any to any discernable degree.

The supportive element may be sonolucent, i.e. made of materials allowing ultrasound waves to penetrate through the supportive element into the underlying mammalian body. In this may, ultrasound can be used to scan the underlying tissue, e.g. the chest cavity including the lungs. This makes it easy to monitor and diagnose any ongoing pathology (e.g. pneumothorax, haemothorax or pneumo-haemothorax) inside the chest cavity. More importantly, it makes it possible to monitor the effect of the drainage treatment by observing the extension of the intrapleural air and amount of fluid inside the chest cavity. A non-sonolucent material (e.g. normal dressings used today) will block the ultrasound waves and make it impossible to monitor the treatment effect.

A flexible layer (18) herein may comprise a flexible or elastic material, such as a rubber material, plastic material, or the like, or a material comprising rubber or plastic providing a similar structure. Non-limiting examples of such materials are polyamide (PA), polyoximetylen (POM), and high molecular polyethylene (HMPE). Said flexible layer may comprise a non-permeable flexible material, a water repellant or water resistant material. The material could also be a fabric with the required characteristics. The flexible layer may also be referred to as the top layer.

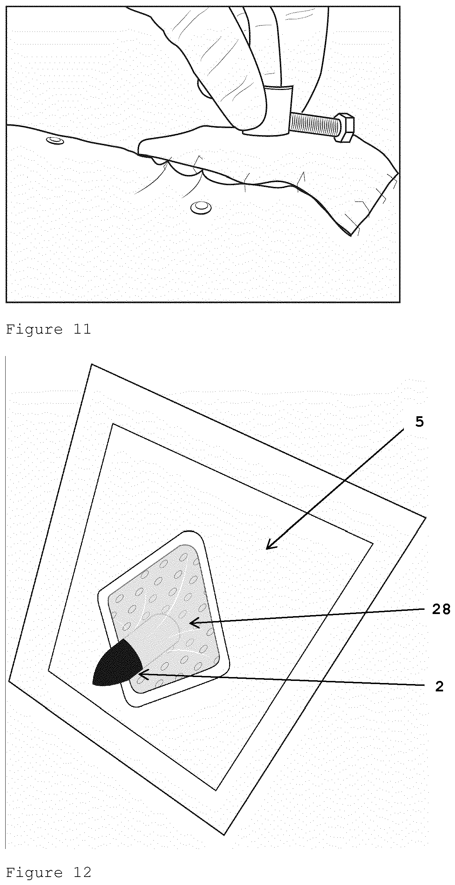

As illustrated in figure 4, said flexible layer (18) may extend beyond at least one of the edges of the ductile layer thereby forming an outer rim (22) of a flexible material around said supportive element (5). Such a rim (22) may be used to attach stitches to the body to which the tube fixation device (1) is attached to, providing a thin material which is easy to attach stitches through. This is also illustrated in figure 9 and 11. The rim (22) may have a width of about 0.5 cm, i.e. the rim extends beyond the edges of the adhesive layer, the adsorbent layer and/or the ductile, bendable layer with about 0.5 cm (see figure 4), but is not limited thereto. Other examples of rim widths are in the range of about 0,2-4 cm, preferably in the range of about 0,4-1,0 cm. Accordingly, the medical staples pass through the rim (22) and attach the device to the skin (see e.g. figure 11 and 9). Further, the rim itself may consist of several layers such as flexible layer, absorbent layer and adhesive layer.

Said layered structure (16) herein may also comprise an absorbent layer (24), said layer comprising an absorbent material. Examples of suitable materials for the absorbent material are also provided herein, but may be any available absorbent material suitable for the purpose. The absorbent material allows for absorbing bodily fluids around the drainage area during the use of the tube fixation device (1). As mentioned above, the ductile layer and the absorbent layer may also be referred herein as forming a dual core. The combination of arranging a ductile layer and an absorbent layer, especially when arranging the ductile layer on top of, or above, or embedded in the absorbent layer, creates a synergy to contain body fluids within the device, and to prevent as much bleeding as possible from the wound site. As mentioned above, the compressive force of the ductile layer works together with the absorbent material in the absorbent layer to effectively prevent bleeding from the wound site.

Said layered structure (16) herein may also comprise an adhesive layer (26), said layer (26) comprising an adhesive material, or dressing. The adhesive layer can also contain a tape or another suitable adhesive. The adhesive layer is the layer of the supportive element that is arranged closest to the body to which the tube fixation device (1) is attached. This layer provides for attachment of the device to the body. As mentioned above, the adhesive layer should preferably be provided with a number of small pores or holes to increase the diffusion of liquids, such as blood and other body fluids, from the wound area to be absorbed into an absorbent layer. This layer may also be referred to as the bottom layer.

Hence, the attachment of the device (1) to the body may be through using both an adhesive layer (e.g. a tape or other suitable adhesive) and stitches through the rim (22). This technique provides an improved and faster technique than conventional methods.

Accordingly, said layered structure (16) may comprise at least four layers in the following consecutive order:
a) an adhesive layer (26);
b) an absorbent layer (24);
c) a ductile, bendable layer (20), such as a metal layer; and
d) a flexible layer (18),
and wherein layer a) is adapted to be arranged in direct contact with a mammalian body as an inner or bottom layer, and wherein layer d) forms the outer or top layer of the layered structure and being arranged with a rim (22) for attaching stitches through the rim (22) of said flexible layer.

Examples of shapes of the layered structure (16) or the supportive element are apparent from the figures. The different layers are preferably of the same shape as each other and cover essentially the same surface area. However, it is also conceivable that some layer cover a smaller area than others. The overall shape of the supportive element is an essentially flat structure, and can be any suitable shape, such as square, rectangular, circular, elliptical etc. As mentioned, when the flexible layer forms a rim, this layer extends beyond the other layers.

The absorbent layer (24) and the ductile bendable layer (20) are also referred to herein as the dual core of the layered structure, e.g. the dual core bendable plate and the dual core absorbent dressing.

One embodiment of the tube fixation device is illustrated in figure 6a, wherein the device (1) may have the following features. The tube receiving element (3) is in the form of a pipe-like structure with a screw or a threaded element and a handle. The tube is to be threaded through the pipe and opening. The screw may be tightened to fasten the drain after insertion. The tube receiving element (3) may prevent kinking of the tube. The device further comprises a layered structure (16) comprising a dual core that comprises two materials - a ductile metal plate that makes the device bendable to better fit to the curvatures of the patient's chest and a (super) absorbing dressing to remove any blood and sweat from the skin. In addition, a paste adhesive dressing underneath the supporting element may further fix the plate to the skin and reduce any leakage of air around the chest tube. This forms the adhesive layer. Additionally, a surgical stapler may stitch the supportive element, e.g. the plate to the patient's chest through the rubber rim that surrounds the tube fixation device (1), (e.g. by the use of a Proximate® Skin Stapler). Accordingly, the rubber rim is a part of the flexible layer as further described herein (see figure 4). Accordingly, an adhesive layer combined with a threaded element such as a screw handle and the stitches through the rubber rim is useful for securing the drain and holding it in place even in challenging situations with increased chance of pulling out the drain (transportation, emergency room examination, ICU-settings etc.)

Figure 6b illustrates a supportive element (5) of an embodiment of the device (1) showing the layered structure (16), as described above.

Figure 8 illustrates another embodiment of the tube fixation device, wherein the tube receiving element (3) and the fastening element (4) together forms a clamp-like structure allowing the tube (2) to be fixated by the fastening element (4) being in the form of a screw-like structure optionally with a handle that when rotated tightens or compresses the tube (2) against the wall of the tube receiving element (see Figure 8b). As seen in figure 8d, the tube fixation device may be provided in an adult size (left) and a pediatric size (right). The tube receiving element (3) and the fastening element (4) may be made of plastic or metal.

In one embodiment, the tube receiving element (3) has a cylindrical, shape, like a pipe, and comprises a metal material, the fastening element (4) comprises a screw of a metal material, the flexible layer (18) comprises a rubber material, such as a rubber band, the absorbent layer (24) comprises a super absorbent dressing, the adhesive layer (26) comprises an adhesive tape, and the ductile layer (20) comprises a metal material. The flexible layer (18) is provided with a rim (22) surrounding the supportive element (5). The rim may have a width in the range of about 0,2-10 cm, preferably about 0.5 cm.

In some embodiments, the tube receiving element (3) is welded, glued, clipped, screwed or similarly fastened to at least one of the layers of the supportive element, e.g. the ductile layer. In such an embodiment, when suitable materials are used in the respective elements, such as a plastic or metal in the tube receiving element (3) and a shapeable but firm metal, plastic or polymer in the ductile layer, the tube receiving element (3) can be angled or bent in relationship to the supportive element (5). Preferably, materials are chosen such that a chosen angle is maintained after bending the tube receiving element (3) in a desired angle. Thus, the chest tube is protected against kinking and it is possible to arrange and rearrange the tube receiving element (3) in a suitable working position on the outside of the patient's body.

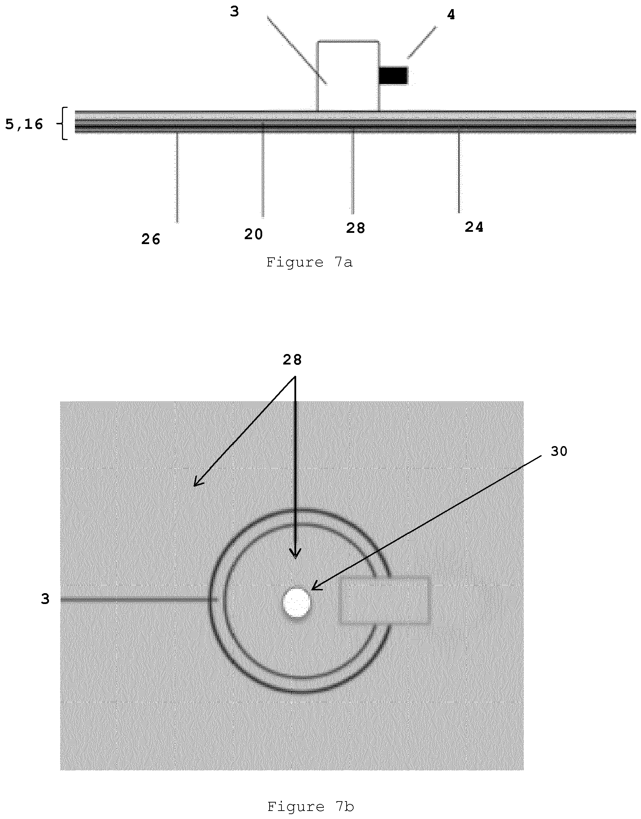

In further embodiments, and as shown in figure 7a, the supportive element (5) of the tube fixation device (1) comprises a sealing layer (28). The sealing layer can preferably be arranged under the ductile layer (20), i.e. closer to a patient's skin than the ductile layer (20). If the supportive layer also comprises an absorbent layer (24), the sealing layer (28) is preferably arranged above the absorbent layer (24), i.e. between the ductile layer (20) and the absorbent layer (24). However, as an alternative, the sealing layer (28) can be arranged under the absorbent layer (not shown in Figure 7a). A flexible layer (18) can be arranged on top of all layers, as shown in Figure 7a and as described above, and can preferably comprise a rim (22) extending beyond the underlying layers (as described for Figure 4).

Preferably, the sealing layer (28) extends over essentially the same surface area as the ductile and/or any absorbent layer, i.e. a majority of the surface area of the supportive element (5), with the addition of also extending inward into the opening for the tube receiving element (3). This is illustrated in figure 7b for one embodiment. Thus, the sealing layer in this embodiment comprises a small opening (30) for a tube (2), wherein the opening (30) for a tube is arranged centrally in the opening for the tube receiving element (3) in the supportive element (5). This small opening (30) for a tube is considerably smaller than the size of the opening for the tube receiving element (3) in the supportive element, and is adapted to be have a diameter that is smaller than a tube to be inserted into the tube receiving element (3).

As an alternative to a small opening in a sealing layer (28) to receive the tip of a tube (2) within the tube receiving element (3), a sealing layer could also be arranged with two or more overlapping flaps of essentially flat material (not shown in the figures) extending into the circumference defined by the tube receiving element, or with a single or cross-shaped slit in a flat material (not shown in the figures), such that a tube can be pushed through the plurality of flaps or through a slit to effectively seal around the tube in a similar way as described above.

It is also conceivable that the sealing layer according to any of the above embodiments does not extend over the entire surface of the supportive element, but instead extends only a certain distance outside the circumference of the tube receiving element, as long as the coverage is enough to provide the sealing features described herein.

The sealing layer (28) comprises a pliable and durable material, and provides the supportive device (5) and thereby the tube fixation device with an additional sealing function over the entire surface of the supportive element against fluids and air leakage from the wound site on the body. Non-limiting examples of materials of the sealing layer are thin rubber or plastic such as latex, poly-urethane, and polyisoprene.

Further, as the sealing layer (28) is provided with an opening (30) of a smaller diameter than the diameter of a tube to be inserted, and being pliable and/or elastic, the sealing layer will seal tight against the outside of a tube inserted therethrough. Thus, the sealing layer preferably provides leakage protection both around the periphery of the wound site, but also around the tube which is inserted into the tube receiving element. The sealing layer also further adds to the maintenance of sterility in the wound area, as bacteria and other contaminants from the outside are also sealed off.

In the embodiments with a sealing layer, a further advantage is also obtained. The sealing layer arranging in the opening for the tube enables an optional pre-loading feature of the device which saves time when applying the tube fixation device. Briefly, a tube can be inserted through the multi-layered structure of the supportive element (5) and the sealing layer (28) will fasten the tube (2) to the fixation device before the tube is to be inserted into the body. This is illustrated in Figure 12, which shows the supportive element from the bottom side, i.e. the side to be applied towards the mammalian body. Thus, a user can preload a device on a chest tube, and when the time comes to apply the device to a patient, the preloading will save time and effort in the application procedure. The preloaded chest tube can be quickly inserted into the patient, and thereafter the fixation device slid down along the tube and placed in position.

As an alternative to, or in addition to, a sealing layer as described above, the tube receiving element can be arranged with a foam material of a suitable kind (not shown in the figures), preferably with a small opening or passage therethrough, such that when a tube is inserted through the tube receiving element, the foam will hold and fill the volume around the tube within the tube receiving element, and effectively hold the tube in place, while providing a sealing function similar to a sealing layer. A pre-loading procedure as described above can be used with such a foam material.

In a further embodiment, the layered structure (16) may comprise at least five layers in the following consecutive order:
a) an adhesive layer (26);
b) an absorbent layer (24);
c) a sealing layer (28);
d) a ductile layer (20); and
e) a flexible layer (18),
   and wherein layer a) is adapted to be arranged in direct contact with a mammalian body as an inner or bottom layer, and wherein layer e) forms the outer or top layer of the layered structure and being arranged with a rim (22) for attaching stitches through the rim (22) of said flexible layer.

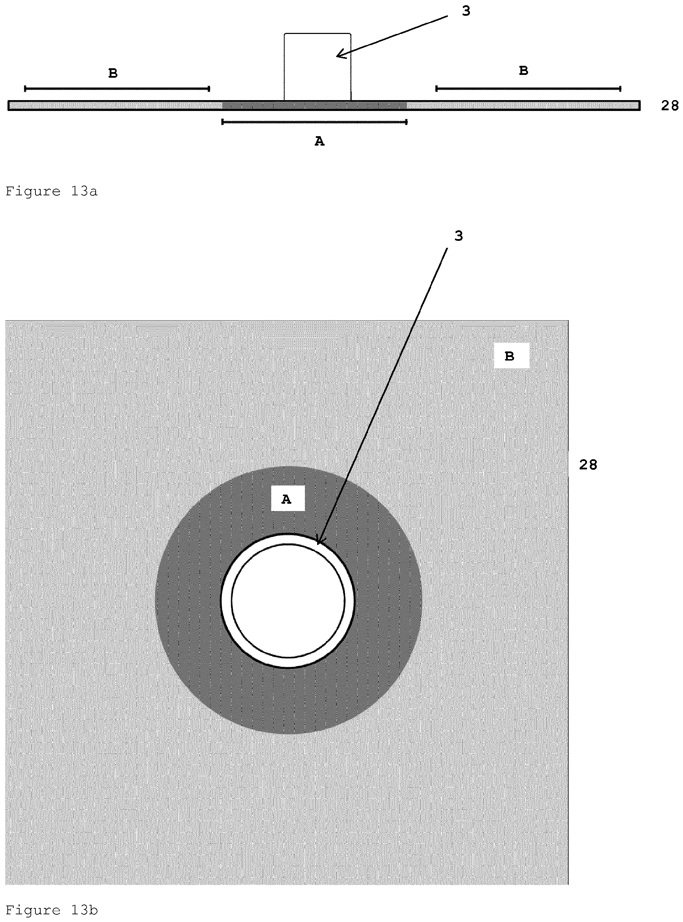

In any embodiment herein, a ductile layer can comprise two different zones with different material characteristics. This is illustrated in Figure 13, where Figure 13a shows a schematic side view of a ductile layer (28) and a tube receiving element (3), and Figure 13b shows a schematic top view of the same. For illustrative purposes other parts of the tube fixation device and the supportive layer are not shown in Figure 13. A first area (zone A) of the ductile layer is arranged around the tube receiving element, preferably with a diameter within the range of about 2-6 cm, more preferably about 3-5 cm. The shape of zone A can be circular, as illustrated in Figure 13b, but can also be any other suitable shape, such as square, rectangular, elliptical, star-shaped etc. Outside zone A, a second area (zone B) is arranged, extending to the periphery of the ductile layer. In some embodiments, zone A comprises a thinner, more compressible or shapeable material, and zone B a more firm and stable material. The two materials can be any suitable material, e.g. metal or plastic. Further, the two zones A and B can comprise the same material, e.g. a metal alloy, but with different thicknesses. The two zones can also be two different materials, e.g. two different metal alloys, or a metal alloy in one zone and a plastic or other material in the others zone.

In an embodiment with two different zones in the ductile layer, the inner zone A allows better adaptability to underlying curvature and also allows for better bending of the tube receiving element. A ductile layer with zone A comprising a more compressible or shapeable material also improves the maintenance of pressure applied directly over the wound site, to effectively lessen bleeding or seeping of fluid. A user can effectively press down harder on the central area of the device, i.e. zone A and the tube receiving element, which can bend in towards the body. The surrounding area of a firmer material, defined by zone B, keeps the supportive element adapted to the chest curvature. The combination of using two different materials as described is an improved adaptability to the patient's body while providing optimal sealing of the wound site combined with optimal wound compression.

Using two different zones in the ductile material can also further enhance the feature of being able to bend the tube receiving element in different directions, as described above. Using a more shapeable material in the inner zone A will enable easier bending and maintenance of a desired angle of the tube receiving element in relation to the mammalian body.

In other embodiments, zone A comprises a transparent material, and zone B a non-transparent material. In such an embodiment, the shapes of the other layers in the supportive element are adapted to the shape of zone B, such that a user can see through the supportive element to the underlying wound site. This enables a user to view the wound site and can monitor e.g. bleeding from the wound, fluid seepage, fluid buildup, infection and other changes. Especially if a transparent tube receiving element is combined with a transparent area of the supportive element surrounding the chest tube receiving element, optimal viewing of the wound site and chest tube is achieved.

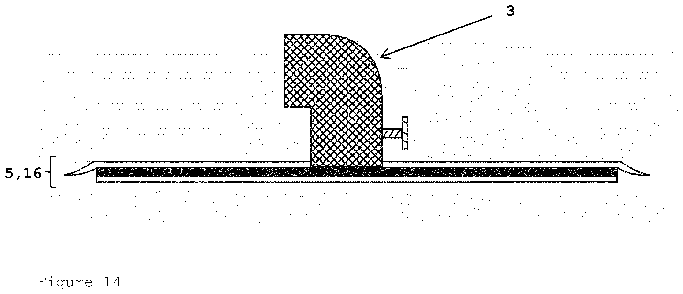

In yet another embodiment, a tube receiving element (3) for a tube fixation device (1) can be arranged to be bendable. This is illustrated in Figure 14, wherein a tube fixation device (1) is shown comprising a supportive element (5) as described in any embodiment herein, and comprising a bendable tube receiving element (3). The tube receiving element (3) can comprise any suitable material as described previously, and is provided with a bendable section. Thus, the tube receiving element can be bent in any desired direction. This embodiment can also be combined with using two different materials in the ductile layer, to provide enhanced possibilities for the user to direct the tube receiving element in a desired direction.

In yet another embodiment, a tube receiving element (3) for a tube fixation device (1) can be arranged to be bendable and possible to rotate (not illustrated). Thus, the tube receiving element can be bent and rotated in any desired direction.

In further embodiments, the ductile layer, or at least a part of the ductile layer, such as a metal part, is arranged in the supportive element such that it can be removed and optionally reinserted or reattached (not shown in the figures). This is useful in situations when an imaging technique, such as CT (Computed Tomography) or X-ray imaging, needs to be used on the patient. In certain imaging procedures, metal materials will disrupt or distort the image and/or cause other problems. In such situations it is an advantage to be able to temporarily or permanently remove the ductile layer, without removing the rest of the chest tube fixation device. In some situations, it may not be necessary to reinsert or reattach the ductile layer after imaging, e.g. if bleeding has lessened or stopped.

Another example of when a removable and optionally re-attachable ductile layer is useful is in very hot or cold climates. For instance, in extreme temperatures, a metal layer can cause burns or freezing injuries to a patient, and can thus not be applied or at least should be removed after a short time period, e.g. when the bleeding has lessened.

In embodiments comprising a removable ductile layer, it may be necessary to arrange the layers of the supportive device in a different order than described above. In one example, a removable ductile layer is arranged as a top layer, i.e. farthest from the skin, to enable easy removal, and attached to underlying layer using an adhesive or glue allowing removal, e.g. a Velcro-fastening or removable tape. In such an embodiment, the ductile layer will still impart compressive forces to the underlying absorbent layer. As an example, the supportive element could comprise in the following consecutive order:
a) an adhesive layer (26);
b) an absorbent layer (24);
c) optionally a sealing layer (28);
d) a flexible layer (18); and
e) a removable ductile layer (20),
   and wherein layer a) is adapted to be arranged in direct contact with a mammalian body as an inner or bottom layer, and wherein layer e) forms the outer or top layer of the layered structure.

In any embodiment above, an anti-infective agent can be comprised within the supportive element. The anti-infective agent can be impregnated into the absorbent material, can be applied as a coating on e.g. the entire of part of the absorbent layer and/or the adhesive layer, or can be comprised in a separate layer, or any combination of the above. Such an anti-infective layer may be arranged between the absorbing layer and adhesive layer. The anti-infective agent will protect against bacterial growth around and in the wound site. Non-limiting examples of anti-infective agents are disinfectants, alcohols, or antibiotics.

In a further embodiment, the layered structure (16) may comprise at least six layers in the following consecutive order:
a) an adhesive layer (26);
b) an anti-infective layer;
c) an absorbent layer (24);
d) a sealing layer (28);
e) a ductile layer (20); and
f) a flexible layer (18),
   and wherein layer a) is adapted to be arranged in direct contact with a mammalian body as an inner or bottom layer, and wherein layer f) forms the outer or top layer of the layered structure and being arranged with a rim (22) for attaching stitches through the rim (22) of said flexible layer.

A tube fixation device (1) as defined herein may be a tube fixation device (1) for a chest tube. Accordingly, encompassed by the present disclosure is a chest tube fixation device (1) containing any structural features as defined herein.

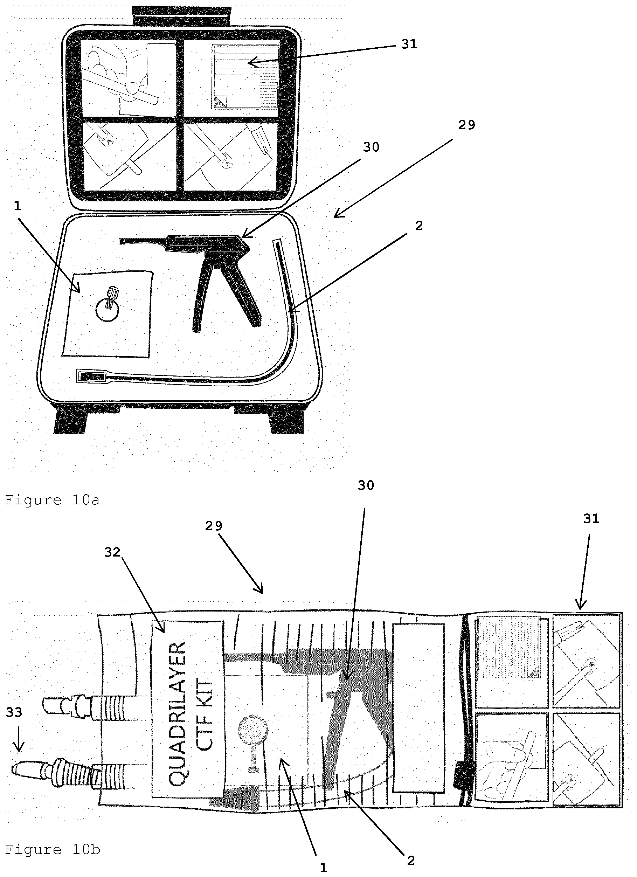

As illustrated in figure 10, the present disclosure also relates to a kit (29) comprising a tube fixation device (1) as defined in any of the aspects or embodiments herein, said kit (29) at least further comprising a device for applying surgical staples (stapler) (30), a tube (2) and instructions for use (31). The tube (2) provided in the kit may be a chest tube. A further illustrated in figure 10a, such a kit (29) may be comprised in a box, such as a sterile box.

In another embodiment, shown in figure 10b, a kit (29) comprising a tube fixation device (1) as defined in any of the aspects or embodiments herein further comprises an outer packaging (32) adapted to contain and enclose components of said kit (29), said packaging (32) being adapted to receive and contain fluids and air in an interior space and wherein said packaging preferably comprises a connection means (33) for connecting said interior space of said packaging (32) to said tube (2). The connection means (33) is preferably provided with a means to connect a suction device, such that suction can be applied to the interior of the bag and thus also to the chest tube when connected to the bag. This will improve drainage through the tube and also make it possible to quickly empty the bag during or after use.

Such an outer packaging can be e.g. a medical collection bag (32), for connection to a tube (2) and collection of drainage fluids and/or air from a wound site or body cavity. Such a bag could be a soft or pliable medical collection bag for fluids. In a preferred embodiment, the components of such a kit (29) are packaged within such a collection bag, which doubles as a container bag for the kit materials.

In such a kit packaged in a medical collection bag (32), it is also conceivable that the bag comprises at least two separate compartments, and that one such compartment is adapted to contain the above described kit components, and the other compartment is adapted for collection of fluids or air, preferably with a connection means for said tube, as described above.

The kit can also further comprise one or several of the following: a scalpel, tweezers, sterile gloves and medical wipes. Preferably the size of a kit comprising the tube fixation device is adapted to a size which can be inserted into a standard clothes pocket of e.g. medical or military personnel, such as ambulance or emergency room personnel, soldiers or field medics.

Preferably a kit (32), contained in a bag as described, can be provided with a Ziploc or similar easy-to-open opening means. This enables ease of use, especially in field or ambulance settings, where the bag can easily be opened and closed to access the components, and more preferably to be able to empty the collected fluids and air during a drainage procedure. Thus, such a bag can then be reused several times.

In another aspect, the present disclosure relates to a method for maintaining a tube (2) inserted into a mammalian body, such as a human body, also referred to as the patient, in a releasably fixated position, comprising arranging said tube (2) into a mammalian body in need of a drainage, and fastening said tube (2) in said tube receiving means (3) of said device (1) through said fastening element (4) of said device (1). The method further may further comprise a step of attaching the supportive element (5) of the device (1) to the mammalian body by using a stapler adding stitches through the rim (22) of the supportive element thereby attaching the device (1) through the supportive element (5) to the skin of the mammalian body. The tube (2) introduced may be a chest tube. Alternatively or additionally, staples or stitches can be applied through multiple layers or all the layers of the supportive element, thereby attaching the device (1) through the supportive element (5) to the skin of the mammalian body.

Figure 9 illustrates an example of the insertion of a drainage tube (2) into the body of a patient and attachment with a tube fixation device (1) as defined herein. One such procedure possible is described in the below:
*Inserting:* First the skin is disinfected using a disinfection solution. Then a chest wall skin incision is made of 2-3 cm in length. Normally the incision is made in the 5-7th intercostal space just laterally to the large pectoral muscle. To penetrate the chest wall scissor and large surgical forceps used to divide deeper muscle layers and finally penetrate the inner pleural layers to enter the chest cavity. A finger is put through the hole to expand the channel where the chest tube will be put. Finally, the chest tube is guided by finger into the chest cavity.
*Fixating with the tube fixation device (1):* An example of a four-step use of the tube fixation device (1) is presented. A) The chest tube is inserted through a skin incision into the chest cavity as described above. B) The tube fixation device (1) is slid over the tube and taped to the skin. The ductile layer, e.g. the metal plate within the core makes it flexible so it can be shaped to better fit the chest wall. The core dressing, i.e. the absorbent layer, will absorb blood and sweat from the skin. The plate closes off the hole through the chest wall so no air will leak around the plastic tube and the compression from the device stop bleeding from the skin incision. C) Staples going through the rubber rim into the skin attaches the dual core plate even more, preventing inadvertent tube removal. D) The chest tube is tightly fastened with the screw mechanism inside the central pipe. The transparency makes it possible to read the length of the chest tube inside the cavity. The protruding pipe also prevents any kinking of the chest tube on the outside of the chest. If unscrewed, the chest tube position may be adjusted. It is also possible to pre-load the device onto the chest tube as described above.

### Examples of materials and dimensions applicable herein

The below are only examples, the present disclosure is not intended to be limited thereto. Any combinations of the below materials may be used.
Flexible layer: The upper layer may be a rubber sheet; a thin layer of rubber that covers the dual core plate (absorbent layer and ductile, bendable layer) with the rim exceeding the four edges of the plate. The device may be pegged to the skin by medical staples going through the rubber rim into the skin. The suggested material is rubber bandage (see table). Another possibility is to use a thin wear proof fabric. The main criteria are that the rubber/fabric should be thin so the medical stables goes through and into the skin.

The medical stapler used in our testing of the prototype is Proximate® Skin Stapler, 35 mm wide.

Dual core material 1 (absorbent layer): Absorbing dressing. An absorbing material inside the core should be used. The preferred solution is a thin super absorber. A lot of different dressings are available (see table 1). Anti-infective chemicals could be imbedded in the absorbing layer.

Dual core material 2 (ductile, bendable layer): Bendable plate to be shaped to the curvatures of the chest wall. In our prototype we used a thin metal plate used in SAM Medical products, www.sammedical.com. This material is a thin sheet of aluminum alloy. The material could also be something else than metal, such as plastic. Examples of different materials than can be uses is shape-retaining plastic material (e.g. PA2200 or a similar flexible plastic polymer)or a bendable and strong metal such as Alumide® or equal bendable metal alloy (reference). The key element is that it is bendable in all directions so that it can be fitted to the shape of the chest and retains its shape as described above.

The adhesive cover (adhesive layer).: a layer of adhesive tape/dressing. There are many different producers on the market.

Non-limiting examples of material in the sealing layer is any suitable thin wearable rubber, plastic or latex.

**Table 1. Non-limiting examples of possible materials for the tube fixation device (4 layers)**

| **Part of the Dual Core Chest Tube Fix** | **Material in our first prototype** | **Other possible materials** | **Producers of such materials** |
|---|---|---|---|
| **Pipe** (tube receiving element (3)) | Metal (e.g. aluminium) | Hard plastic, wear-proof fabric | . |
| **Screw** (fastening element (4) | Metal (e.g. aluminium) | Hard plastic | . |
| **Thread/strips** | Not used in our prototype | Thread and plastic strips | |
| **Rubber/plastic** (flexible layer (18)) | Rubber | Thin wear proof fabric | 1) VBM Meizintechnik GmbH, Germany |
| **Dual core** - dressing (absorbent layer (24)) | 1) Superabsorbent wound dressing | Other absorbent dressings such as | 1) Curea Medical GmbH, Germany. www.curea-medical.de/ueber-uns/ |
| | | | 2) OneMed. Finland. www.onemed.no |
| | | 2) Gauze Swabs | 3) OneMed. Finland. www.onemed.no |
| | | 3) Absorbent dressing Evercare | 4) Molnlycke Health Care, Sweden. www.molnlycke.no |
| | | 4) Mesoft. | 5) Bastos Viegas, Portugal, www.onemed.no |
| | | 5) Non-adherent dressing | 6) Shaoxing Zhengde Surgical Dressing Co., Ltd. China. http://www.zhende.com |
| | | 6) Compresses from Zhende | |
| **Dual core flexible/bendable plate** (ductile, bendable layer (20)) | 1) Metal, aluminium alloy | Other bendable/flexible materials; Plastic | 1) Same metal used inside the SAM splint from Samedical. Norwegian supplier is Ferno Norden, Examples as described above |
| **Sealing layer** | Not applicable | Thin strong rubber membrane | 1. Same rubber as in condoms |
| | | | 2. Same membrane as in Prometheus Medical's chest seal product. |
| | | | 3. Examples of materials of the sealing layer are thin rubber or plastic such as latex, polyurethane, polyisoprene. |
| **Adhesive tape** (adhesive layer (26)) | Different tape solutions | Any adhesive tape | E.g: Allevyn Adhesive dressing from Smith and Nephew. England. Tape from underneath defibrillation pads to LifePack 15. |

The following are non-limiting examples of dimensions applicable to the tube fixation device (1).

### Suggested dimensions of the tube fixation device (1):

1) Adult square version
   Dimensions dual core plate: (8 ± 4 cm) x (8 ± 4 cm)
   Pipe height: 2 ± 1.0 cm
   Pipe diameter: 1.5 - 3 cm
   Thickness supportive element less than 1.0 cm, ductile layer 0.1-0.4 cm Weight less than 100 gram
2) Pediatric square version:
   Dimensions dual core plate: (4 ± 2 cm) x (4 ± 2 cm)
   Pipe height: 1 ± 1.0 cm
   Pipe diameter: 0.5 - 1 cm
   Thickness supportive element less than 1.0 cm, ductile layer 0.1-0.4 cm
   Weight less than 50 gram
   We could also consider a round version.
3) Adult round version
   Diameter dual core plate: (8 ± 4 cm)
   Pipe height: 2 ± 1.0 cm
   Pipe diameter: 1.5 - 3 cm
   Thickness supportive element less than 1.0 cm, ductile layer 0.1-0.4 cm
   Weight less than 100 gram
4) Pediatric round version:
   Diameter dual core plate: (4 ± 2 cm)
   Pipe height: 1 ± 1.0 cm
   Pipe diameter: 0.5 - 2 cm
   Thickness supportive element less than 1.0 cm, ductile layer 0.1-0.4 cm
   Weight less than 50 gram

### EXPERIMENTAL SECTION

A prototype of a tube fixation device as described herein has been tested on anesthetized pigs in an experimental animal laboratory called Sandnes Education And Research Centre Høyland (SEARCH). The highlights of the protocol that we used:
- Two pigs where anesthetized.
- One chest tube was inserted on the left side using a standard surgical set and fixated with sutures.
- One chest tube was inserted on the opposite right side with a surgical set and fixated with our multi-layer Chest Tube Fix.
- The time trial measurement A) We measured the time from skin incision to the completion of tube fixation on the left side (sutures) and compared the time with the right side (multi- layer Chest Tube Fix). The results are shown below.
- We then performed a "pull" test by pulling the Dual Core Chest Tube Fix as shown in the picture B). That was just to see how much force that was needed before the device detached from the skin. This test may be more objective in later prototype testing, by attaching increasing number of weights to the chest tube. Different anchoring methods should be compared in the future.

### Time from skin incision to chest tube fixation: Results of the trials at SEARCH

| | |
|---|---|
| Normal suture technique | 4 min 5 sec |
| Multi-layer chest tube fix | 1 min 25 sec |

Above, the tube fixation device and corresponding method of use has been described with reference to specific embodiments. It is however obvious to a person skilled in the art that other embodiments may be used to achieve the same results within the scope of the invention as defined by the claims.

## Claims

1. A tube fixation device (1) for maintaining a tube (2) inserted into a mammalian body in a releasably fixated position, said tube fixation device (1) comprising:
a tube receiving element (3) configured to receive said tube (2);
a fastening element (4) arranged in connection to said tube receiving element (3) and wherein said fastening element (4) is configured to releasably fixate said tube (2) when said tube (2) is arranged in said tube receiving element (3); and
a supportive element (5) attached to said tube receiving element (3) and configured to be positioned on a surface area of said mammalian body, wherein the supportive element (5) comprises a layered structure (16),
**characterized in that** said layered structure comprises at least a ductile layer (20) comprising a ductile material, and an adhesive layer (26) comprising an adhesive material, wherein the ductile layer is configured to conform to a three-dimensional shape of said surface area on said mammalian body when pressure is applied to the supportive element (5).

2. A tube fixation device (1) according to claim 1, wherein said ductile layer is further configured to essentially maintain said obtained three-dimensional shape of said surface area on said mammalian body.

3. A tube fixation device (1) according to any preceding claim, wherein the ductile material comprises a shape-retaining plastic material, a plastic polymer, a metal and/or an alloy.

4. A tube fixation device (1) according to any preceding claim, wherein said ductile layer, or at least a part of said ductile layer, is adapted to be removed and optionally reinserted or reattached in said supportive element.

5. A tube fixation device (1) according to any preceding claim, wherein said ductile layer comprises at least two zones comprising different materials, wherein one of said materials defines a first area or zone (A) arranged centrally around said tube receiving element, and the second of said materials defines a second area or zone (B) arranged more peripherally in said ductile layer.

6. A tube fixation device (1) according to any preceding claim, wherein said layered structure further comprises an absorbent layer (24) comprising an absorbent material, wherein said absorbent layer (24) is arranged below or under said ductile layer (20) in relation to said surface area of said mammalian body.

7. A tube fixation device (1) according to any preceding claim, wherein said layered structure further comprises an absorbent layer (24) comprising an absorbent material, wherein said absorbent layer (24) is arranged above or over said ductile layer (20) in relation to said surface area of said mammalian body.

8. A tube fixation device (1) according to any preceding claim, wherein said layered structure (16) comprises a flexible layer (18) comprising a flexible material, wherein said flexible layer (18) is arranged above or over said ductile layer (20) in relation to said surface area of said mammalian body.

9. A tube fixation device (1) according to claim 8, wherein said flexible layer (18) extends beyond at least one of the edges of the ductile layer (20), thereby forming a rim (22) of a flexible material around at least a part of said supportive element (5).

10. A tube fixation device (1) according to any preceding claim, wherein said adhesive layer (26) is arranged closest to said surface area of said mammalian body, to provide adhesion of said supportive element (5) to said mammalian body.

11. A tube fixation device (1) according to any one of claims 6-10, wherein said tube fixation device (1) comprises a sealing material adapted to essentially seal a space surrounding said tube (2) when said tube (2) is arranged in said tube receiving element (3) from any leakage of fluid or air.

12. A tube fixation device (1) according to claim 11, wherein said sealing material is arranged inside the tube receiving element (28).

13. A tube fixation device (1) according to claim 11 or 12, wherein said sealing material is a sealing layer (28) within said layered structure (16).

14. A tube fixation device (1) according to claim 13, wherein said sealing layer (28) is arranged between said ductile layer (20) and said absorbent layer (24).

15. A tube fixation device (1) according to any preceding claim, wherein said fastening element (4) is configured to apply a radially directed fastening pressure in relation to said tube (2).

16. A tube fixation device (1) according to any preceding claim, wherein said fastening element (4) comprises an elongated threaded element (6) and wherein said tube receiving element contains a threaded opening (8) adapted to receive and rotatably attach said threaded element (6) to said tube receiving element (3).

17. A tube fixation device (1) according to any one of claims 1-14, wherein said fastening element (4) is configured to apply a partially or fully circumferential fastening pressure in relation to said tube (2).

18. A tube fixation device (1) according to any preceding claim, wherein said tube receiving element (3) is an elongated hollow pipe (3).

19. A tube fixation device (1) according to any preceding claim, wherein said layered structure comprises at least four layers in the following consecutive order:
a) an adhesive layer (26);
b) an absorbent layer (24);
c) said ductile layer (20); and
d) a flexible layer (18), and wherein layer a) is adapted to be arranged in direct contact with a mammalian body as an inner or bottom layer, and wherein layer d) forms the outer or top layer of the layered structure and being arranged with a rim (22) for attaching stitches or staples through the rim (22) of said flexible layer.

20. A tube fixation device (1) according to any one of the preceding claims, wherein said tube (2) is a chest tube.

21. A kit (29) comprising a tube fixation device (1) according to any of claims 1-20, said kit further comprising a device for applying surgical staples (30), a chest tube (2) and instructions for use (31).

22. A kit (29) according to claim 21, said kit further comprising an outer packaging (32) adapted to contain and enclose components of said kit (29), said packaging (32) being adapted to receive and contain fluids or air in an interior space and wherein said packaging comprises a connection means (33) for connecting said interior space of said packaging (32) to said tube (2).

## Patentansprüche

1. Schlauchfixierungsvorrichtung (1) zum Aufrechterhalten eines in einem Säugetierkörper in einer lösbar befestigten Position eingesetzten Schlauchs (2), wobei die Schlauchfixierungsvorrichtung (1) Folgendes umfasst:
ein Schlauchaufnahmeelement (3), das konfiguriert ist, um den Schlauch (2) aufzunehmen;
ein Befestigungselement (4), das in Verbindung mit dem Schlauchaufnahmeelement (3) angeordnet ist, und wobei das Befestigungselement (4) konfiguriert ist, um den Schlauch (2) lösbar zu fixieren, wenn der Schlauch (2) in dem Schlauchaufnahmeelement (3) angeordnet ist; und
ein Stützelement (5), das an dem Schlauchaufnahmeelement (3) angeordnet ist und konfiguriert ist, um auf einer Oberfläche des Säugetierkörpers positioniert zu sein, wobei das Stützelement (5) eine Schichtstruktur (16) umfasst,
**dadurch gekennzeichnet, dass** die Schichtstruktur mindesten eine dehnbare Schicht (20), umfassend ein dehnbares Material, und eine haftende Schicht (26), umfassend ein haftendes Material, umfasst, wobei die dehnbare Schicht konfiguriert ist, um mit einer dreidimensionalen Form der Oberfläche auf dem Säugetierkörper übereinzustimmen, wenn Druck auf das Stützelement (5) angewendet wird.

2. Schlauchfixierungsvorrichtung (1) nach Anspruch 1, wobei die dehnbare Schicht weiter konfiguriert ist, um die erhaltene dreidimensionale Form der Oberfläche auf dem Säugetierkörper im Wesentlichen aufrechtzuerhalten.

3. Schlauchfixierungsvorrichtung (1) nach einem der vorstehenden Ansprüche, wobei das dehnbare Material ein formbeständiges Kunststoffmaterial, ein Kunststoffpolymer, ein Metall und/oder eine Legierung umfasst.

4. Schlauchfixierungsvorrichtung (1) nach einem der vorstehenden Ansprüche, wobei die dehnbare Schicht oder mindestens ein Teil der dehnbaren Schicht angepasst ist, um entfernt zu werden und wahlweise in das Stützelement erneut eingesetzt oder daran erneut angebracht zu werden.

5. Schlauchfixierungsvorrichtung (1) nach einem der vorstehenden Ansprüche, wobei die dehnbare Schicht mindestens zwei Zonen umfasst, die unterschiedliche Materialien umfassen, wobei eines der Materialien eine erste Fläche oder Zone (A) definiert, die zentral um das Schlauchaufnahmeelement angeordnet ist, und das zweite der Materialien eine zweite Fläche oder Zone (B) definiert, die in der dehnbaren Schicht peripherer angeordnet ist.

6. Schlauchfixierungsvorrichtung (1) nach einem der vorstehenden Ansprüche, wobei die Schichtstruktur weiter eine absorbierende Schicht (24) umfasst, die ein absorbierendes Material umfasst, wobei die absorbierende Schicht (24) unterhalb oder unter der dehnbaren Schicht (20) in Bezug auf die Oberfläche des Säugetierkörpers angeordnet ist.

7. Schlauchfixierungsvorrichtung (1) nach einem der vorstehenden Ansprüche, wobei die Schichtstruktur weiter eine absorbierende Schicht (24) umfasst, die ein absorbierendes Material umfasst, wobei die absorbierende Schicht (24) oberhalb oder über der dehnbaren Schicht (20) in Bezug auf die Oberfläche des Säugetierkörpers angeordnet ist.

8. Schlauchfixierungsvorrichtung (1) nach einem der vorstehenden Ansprüche, wobei die Schichtstruktur (16) eine flexible Schicht (18) umfasst, die ein flexibles Material umfasst, wobei die flexible Schicht (18) oberhalb oder über der dehnbaren Schicht (20) in Bezug auf die Oberfläche des Säugetierkörpers angeordnet ist.

9. Schlauchfixierungsvorrichtung (1) nach einem der vorstehenden Ansprüche, wobei sich die flexible Schicht (18) über mindestens einen der Ränder der dehnbaren Schicht (20) hinaus erstreckt, wodurch eine Einfassung (22) aus einem flexiblen Material um mindestens einen Teil des Stützelements (5) gebildet wird.

10. Schlauchfixierungsvorrichtung (1) nach einem der vorstehenden Ansprüche, wobei die haftende Schicht (26) am nächsten zu der Oberfläche des Säugetierkörpers angeordnet ist, um Haftung des Stützelements (5) an dem Säugetierkörper bereitzustellen.

11. Schlauchfixierungsvorrichtung (1) nach einem der Ansprüche 6-10, wobei die Schlauchfixierungsvorrichtung (1) ein Dichtungsmaterial umfasst, das angepasst ist, um einen den Schlauch (2) umgebenden Raum gegen ein Austreten von Fluid oder Luft im Wesentlichen abzudichten, wenn der Schlauch (2) in dem Schlauchaufnahmeelement (3) angeordnet ist.

12. Schlauchfixierungsvorrichtung (1) nach Anspruch 11, wobei das Dichtungsmaterial im Inneren des Schlauchaufnahmeelements (28) angeordnet ist.

13. Schlauchfixierungsvorrichtung (1) nach Anspruch 11 oder 12, wobei das Dichtungsmaterial eine Dichtungsschicht (28) in der Schichtstruktur (16) ist.

14. Schlauchfixierungsvorrichtung (1) nach Anspruch 13, wobei die Dichtungsschicht (28) zwischen der dehnbaren Schicht (20) und der absorbierenden Schicht (24) angeordnet ist.

15. Schlauchfixierungsvorrichtung (1) nach Anspruch 13, wobei das Befestigungselement (4) konfiguriert ist, um einen radial ausgerichteten Befestigungsdruck in Bezug auf den Schlauch (2) anzuwenden.

16. Schlauchfixierungsvorrichtung (1) nach einem der vorstehenden Ansprüche, wobei das Befestigungselement (4) ein längliches, mit einem Gewinde versehenes Element (6) umfasst und wobei das Schlauchaufnahmeelement eine mit einem Gewinde versehene Öffnung (8) enthält, die angepasst ist, um das mit einem Gewinde versehene Element (6) aufzunehmen und drehbar an dem Schlauchaufnahmeelement (3) anzubringen.

17. Schlauchfixierungsvorrichtung (1) nach einem der Ansprüche 1-14, wobei das Befestigungselement (4) konfiguriert ist, um einen teilweisen oder vollständigen Umfangsbefestigungsdruck in Bezug auf den Schlauch (2) anzuwenden.

18. Schlauchfixierungsvorrichtung (1) nach einem der vorstehenden Ansprüche, wobei das Schlauchaufnahmeelement (3) ein längliches Hohlrohr (3) ist.

19. Schlauchfixierungsvorrichtung (1) nach einem der vorstehenden Ansprüche, wobei die Schichtstruktur mindestens vier Schichten in der folgenden konsekutiven Reihenfolge umfasst:
a) eine haftende Schicht (26);
b) eine absorbierende Schicht (24);
c) eine dehnbare Schicht (20); und
d) eine flexible Schicht, und wobei Schicht a) angepasst ist, um in direktem Kontakt mit einem Säugetierkörper als eine innere oder untere Schicht angeordnet zu sein, und wobei Schicht d) die äußere oder obere Schicht der Schichtstruktur bildet und mit einer Einfassung (22) zum Anbringen von Stichen oder Klammern durch die Einfassung (22) der flexiblen Schicht hindurch angeordnet ist.

20. Schlauchfixierungsvorrichtung (1) nach einem der vorstehenden Ansprüche, wobei der Schlauch (2) ein Thoraxschlauch ist.

21. Kit (29), das eine Schlauchfixierungsvorrichtung (1) nach einem der Ansprüche 1-20 umfasst, wobei das Kit weiter eine Vorrichtung zum Anwenden chirurgischer Klammern (30), einen Thoraxschlauch (2) und eine Gebrauchsanleitung (31) umfasst.

22. Kit (29) nach Anspruch 21, wobei das Kit weiter eine äußere Verpackung (32) umfasst, die angepasst ist, um Bestandteile des Kits (29) zu enthalten und zu umschließen, wobei die Verpackung (32) angepasst ist, um Fluide oder Luft in einem Innenraum aufzunehmen und zu enthalten, und wobei die Verpackung ein Verbindungsmittel (33) zum Verbinden des Innenraums der Verpackung (32) mit dem Schlauch (2) umfasst.

## Revendications

1. Dispositif de fixation de tube (1) pour maintenir un tube (2) inséré dans le corps d'un mammifère dans une position fixée de manière amovible, ledit dispositif de fixation de tube (1) comprenant :
un élément de réception de tube (3) configuré pour recevoir ledit tube (2) ;
un élément de prise (4) relié à l'élément de réception de tube (3) et dans lequel ledit élément de prise (4) est configuré pour fixer de manière amovible ledit tube (2) lorsque ledit tube (2) est agencé dans ledit élément de réception de tube (3) ; et
un élément support (5) attaché au dit élément de réception de tube (3) et configuré pour être disposé sur une zone de surface dudit corps de mammifère, dans lequel l'élément de support (5) comprend une structure stratifiée (16),
**caractérisé en ce que** ladite couche stratifiée comprend au moins une couche ductile (20) comprenant un matériau ductile, et une couche adhésive (26) comprenant un matériau adhésif, dans lequel la couche ductile est configurée pour se conformer à une forme tridimensionnelle de ladite zone de surface sur ledit corps de mammifère lorsqu'une pression est appliquée à l'élément de support (5).

2. Dispositif de fixation de tube (1) selon la revendication 1, dans lequel ladite couche ductile est en outre configurée pour sensiblement maintenir ladite forme tridimensionnelle obtenue de ladite zone de surface sur ledit corps de mammifère.

3. Dispositif de fixation de tube (1) selon l'une quelconque des revendications précédentes, dans lequel le matériau ductile comprend un matériau de plastique à mémoire de forme, un matériau polymère, un métal et/ou un alliage.

4. Dispositif de fixation de tube (1) selon l'une quelconque des revendications précédentes, dans lequel ladite couche ductile, ou au moins une partie de ladite couche ductile, est adaptée à être retirée et facultativement réinsérée ou refixée dans ledit élément de support.

5. Dispositif de fixation de tube (1) selon l'une quelconque des revendications précédentes, dans lequel ladite couche ductile comprend au moins deux zones comprenant des matériaux différents, dans lequel l'un desdits matériaux définit une première aire ou zone (A) agencée de manière centrale autour dudit élément de réception de tube, et le second desdits matériaux définit une seconde aire ou zone (B) agencée de manière plus périphérique dans ladite couche ductile.

6. Dispositif de fixation de tube (1) selon l'une quelconque des revendications précédentes, dans lequel ladite structure stratifiée comprend en outre une couche absorbante (24) comprenant un matériau absorbant, dans lequel ladite couche absorbante (24) est agencée au-dessous de ou sous ladite couche ductile (20) par rapport à ladite zone de surface dudit corps de mammifère.

7. Dispositif de fixation de tube (1) selon l'une quelconque des revendications précédentes, dans lequel ladite structure stratifiée comprend en outre une couche absorbante (24) comprenant un matériau absorbant, dans lequel ladite couche absorbante (24) est agencée au-dessus de ou sur ladite couche ductile (20) par rapport à ladite zone de surface dudit corps de mammifère.

8. Dispositif de fixation de tube (1) selon l'une quelconque des revendications précédentes, dans lequel ladite structure stratifiée (16) comprend une couche flexible (18) comprenant un matériau flexible, dans lequel ladite couche flexible (18) est agencée au-dessus de ou sur ladite couche ductile (20) par rapport à ladite zone de surface dudit corps de mammifère.

9. Dispositif de fixation de tube (1) selon la revendication 8, dans lequel ladite couche flexible (18) s'étend au-delà d'au moins l'un des bords de la couche ductile (20), formant ainsi un bord (22) d'un matériau flexible autour d'au moins une partie dudit élément de support (5).

10. Dispositif de fixation de tube (1) selon l'une quelconque des revendications précédentes, dans lequel ladite couche adhésive (26) est agencée le plus proche de ladite zone de surface dudit corps de mammifère, pour permettre l'adhésion dudit élément de support (5) au dit corps de mammifère

11. Dispositif de fixation de tube (1) selon l'une quelconque des revendications 6 à 10, dans lequel ledit dispositif de fixation de tube (1) comprend un matériau d'étanchéité adapté à sensiblement rendre hermétique à toute fuite de fluide ou d'air un espace entourant ledit tube (2) lorsque ledit tube (2) est agencé dans ledit élément de réception de tube (3).

12. Dispositif de fixation de tube (1) selon l'une la revendication 11, dans lequel ledit matériau d'étanchéité est agencé à l'intérieur de l'élément de réception de tube (28).

13. Dispositif de fixation de tube (1) selon la revendication 11 ou 12, dans lequel ledit matériau d'étanchéité est une couche d'étanchéité (28) à l'intérieur de ladite structure stratifiée (16).

14. Dispositif de fixation de tube (1) selon la revendication 13, dans lequel ladite couche d'étanchéité (28) est agencée entre ladite couche ductile (20) et ladite couche absorbante (24).

15. Dispositif de fixation de tube (1) selon l'une quelconque des revendications précédentes, dans lequel ledit élément de prise (4) est configuré pour appliquer une pression de prise radialement dirigée par rapport au dit tube (2).

16. Dispositif de fixation de tube (1) selon l'une quelconque des revendications précédentes, dans lequel ledit élément de prise (4) comprend un élément fileté allongé (6) et dans lequel ledit élément de réception de tube contient une ouverture filetée (8) adaptée à recevoir et à lier de manière rotative ledit élément fileté (6) au dit élément de réception de tube (3).

17. Dispositif de fixation de tube (1) selon l'une quelconque des revendications 1 à 14, dans lequel ledit élément de prise (4) est configuré pour appliquer une pression de prise partiellement ou totalement circonférentielle par rapport au dit tube (2).

18. Dispositif de fixation de tube (1) selon l'une quelconque des revendications précédentes, dans lequel ledit élément de réception de tube (3) est un tuyau creux allongé (3).

19. Dispositif de fixation de tube (1) selon l'une quelconque des revendications précédentes, dans laquelle ladite structure stratifiée comprend au moins quatre couches dans l'ordre consécutif suivant :
a) une couche adhésive (26) ;
b) une couche absorbante (24) ;
c) ladite couche ductile (20) ; et
d) une couche flexible (18), et dans laquelle la couche a) est adaptée à être agencée en contact direct avec un corps de mammifère sous la forme d'une couche intérieure ou inférieure, et dans lequel la couche d) forme la couche extérieure ou supérieure de la structure stratifiée et étant agencée avec un bord (22) pour attacher des sutures ou des agrafes dans le bord (22) de ladite couche flexible.

20. Dispositif de fixation de tube (1) selon l'une quelconque des revendications précédentes, dans lequel ledit tube (2) est un tube thoracique.

21. Kit (29) comprenant un dispositif de fixation de tube (1) selon l'une quelconque des revendications 1 à 20, ledit kit comprenant en outre un dispositif d'application d'agrafes chirurgicales (30), un tube thoracique (2) et des instructions d'utilisation (31).

22. Kit (29) selon la revendication 21, ledit kit comprenant en outre un emballage extérieur (32) adapté à contenir et renfermer des composants dudit kit (29), ledit emballage (32) étant adapté à recevoir et contenir des fluides ou de l'air dans un espace intérieur et dans lequel ledit emballage comprend un moyen de raccordement (33) pour raccorder ledit espace intérieur dudit emballage (32) au dit tube (2).
